# EUROPEAN PATENT APPLICATION

(11) **EP 1 048 290 A2**
(43) Date of publication of application: **02.11.2000**
(21) Application number: 00201491.8
(22) Date of filing: 25.04.2000
(51) Int. Cl.: A61K 7/13

(54) **Oxidative hair dye compositions with conditioning and enhanced color deposition**

(30) Priority: 30.04.1999 US 302394
(71) Applicant: Bristol-Myers Squibb Company, New York, N.Y. 10154 (US)
(72) Inventor: Azizova, Marina, Rowayton, Connecticut 06853 (US); Murphy, Bryan P., Monroe, Connecticut 06468 (US); Pohl, Stanley, Scarsdale, New York (US); DeMarco, Richard, Danbury, Connecticut 06811 (US)
(74) Representative: Mays, Julie

(57) **Abstract**

This invention concerns oxidative, hair coloring compositions which color and condition hair, requiring lesser amounts of dye concentrations due to the presence of certain amphoteric, quaternized, conditioning polymers. The polymers contain repeating units from acrylic acid and methacrylamidopropyl trimethyl ammonium chloride, dimethyl diallyl ammonium chloride, or a mixture thereof.

## Description

### FIELD OF INVENTION

This invention relates to oxidative, hair coloring compositions which color and condition hair, requiring minimal dye concentrations.

### BACKGROUND OF INVENTION

Modern hair dyeing methodology has developed from its initiation in the 1950's to the point where, following shampoos and conditioners, today it is the third largest product type in the hair care category.

The most commonly used method of dyeing hair, particularly human hair, is oxidative dyeing in which a mixture of essentially colorless aromatic compounds, generally diaminobenzenes, dihydroxybenzenes and aminophenols, are converted by chemical reactions, that are well known to those skilled in the art, to a blend of colored compounds within the hair fibers. Shortly before use, the colorless aromatic compounds, in a suitable base formulation, are normally mixed with hydrogen peroxide or other strong oxidizing agents. The colored compounds or dyes are typically formed by oxidative coupling between primary intermediates (usually p-phenylenediamines or p-aminophenols) and couplers which are phenols, resorcinols, m-aminophenols or related cyclic compounds. Various shades are developed by using mixtures containing more than one of both the intermediate and the coupler.

Because of their low molecular weights and water solubility the primary intermediates and couplers diffuse easily into the hair where the coupling reaction takes place. The colored products developed by oxidation remain trapped in the hair because of their higher molecular weights, relative insolubility in water and absorptive affinity to the internal hair surface. This is the basis for permanent tints and toners which ideally last for the life of the hair and are relatively unaffected by light, shampooing and perspiration.

The practice of oxidative hair coloring is well known. Typically, it involves the use of a two-part system. One part, the dye component, contains at least one primary intermediate and at least one coupler. Before use, the dye component is mixed with a second part, which is a developer formulation containing an oxidizing agent. The developer oxidizes the primary intermediate to a quinone imine. This, in turn, reacts with a coupler to form a colored compound.

### SUMMARY OF THE INVENTION

It has been discovered by the inventors that certain quaternized polymers that can be used as conditioners in a hair color formulation actually make the dyeing more efficient. They make the color deposit heavier and darker with the same amount of dye. Conversely these polymers allow the same shade to be matched with a lesser amount of dye. This allows for reduction in the cost of a formula and reduces the exposure of consumers to these dyes, some of which are allergens. Accordingly, the present invention is directed to a dye component of a hair coloring composition comprising a mixture of:
(A) an amphoteric polymer comprising repeating units from
   (a) acrylic acid, and
   (b) a cationic monomer selected from the group consisting of: methacrylamidopropyl trimethyl ammonium chloride, dimethyl diallyl ammonium chloride; and a mixture thereof, wherein the molar ratio of (a):(b) is 1:3 or greater;
(B) an oxidative dye primary intermediate; and
(C) an oxidative dye coupler.

The amphoteric polymer in the composition of the present invention is a conditioning, quaternized, amphoteric polymer.

The present invention is also directed to a hair coloring composition comprising the dye component of the invention and a developer component. In addition, the present invention includes a kit comprising at least two containers, one container containing the dye component of the invention and another container containing a developer component. The present invention further is directed to a method comprising applying the oxidative dye composition of the invention to hair, allowing the composition to remain in contact with the hair until a desired hair color has been attained, and rinsing excess dye composition from the hair.

### DETAILED DESCRIPTION OF THE INVENTION

There are a number of primary intermediates and couplers that are used in the practice of oxidative dyeing of hair and can be used in the composition of the invention. Based on their frequency of use, the most useful primary intermediates, are the following: p-phenylenediamine, p-toluenediamine, N,N-bis (2-hydroxyethyl)-p-phenylenediamine, p-amino-phenol, 2-chloro-p-phenylenediamine, 2-methoxy-p-phenylene-diamine, 4-amino-m-cresol, 2-hydroxyethyl-p-phenylenediamine, 1-(2,5-diaminophenyl)-1,2 ethane-1, 2-diol, 1-(5-amino-2-hydroxyphenyl)ethane-1,2 diol, and mixtures thereof. The most useful couplers, based on their frequency of use, are the following: resorcinol, 1-napthol, 2-methylnaphthol, 2-methyl-resorcinol, m-aminophenol, 2,4-diaminophenolethanol, 5-methyl-3-aminophenol, 4-methyl-3-aminophenol and m-phenylenediamine. Many combinations of primary intermediates and couplers are known and described. The following United States patents contain some representative examples:

| | | |
|---|---|---|
| 5,032,138; | | 5,393,305; |
| 5,344,463; | | 5,609,651: |
| 5,393,305; | and | 5,843,193; |

Although hydrogen peroxide is the most widely employed oxidant, other oxidizing agents are known and sometimes used, and can be used in the present invention. These include alkali and alkaline earth metal chlorites, which are described in U.S. Patent 5,032,138. Other oxidizing agents employed in oxidative hair coloring and are useful herein include urea peroxide, melamine peroxide and perborates and percarbonates such as sodium perborate and sodium percarbonate.

All of the patents specifically referenced herein are incorporated herein by reference in their entirety.

The particular conditioning polymers that have been found to be useful in the present invention to enhance dye color are amphoteric polymers which are copolymers of polyacrylic acid and a cationic monomer such as dimethyldiallyl ammonium chloride (DMDAAC) or methacrylamidopropyl trimethyl ammonium chloride (MAPTAC), as well as polymers containing more than two types of monomers, preferably terpolymers, containing two or more of these moieties optionally along with a nonionic monomer. Nonionic monomers useful herein which can be used to prepare the amphoteric polymer of the invention are preferably nonionic derivatives of acrylic acid. Preferred nonionic monomers are acrylic acid esters and acrylamide; preferred acrylic acid esters are C₁-C₄ esters, most preferably methyl acrylate. Examples of amphoteric polymers which are commercially available include Polyquaternium 22 (Merquat 280 and Merquat 295, Calgon Inc.), Polyquaternium 47(Merquat 2001, Calgon Inc.), Polyquaternium 7 (Merquat 550, Calgon Inc.) and Polyquaternium 39 (Merquat 3330, Calgon Inc.). Of those polymers that are useful herein, those that work best have high ratios of acrylic acid to quaternary group (or cationic monomer) in the molecule. Thus, Merquat 2001 is more effective at enhancing dye uptake than Merquat 3330. Polymers that are not effective and are not useful in the present invention have a molar ratio of acrylic acid to cationic monomer of less than 1:3. Thus, the molar ratio of acrylic acid monomer to cationic monomer in the amphoteric polymer of the invention is 1:3 or greater, preferably 1:2 or greater, more preferably 1:1.5 or greater, even more preferably 1:1 or greater. Examples of amphoteric polymers that have a molar ratio of acrylic acid to cationic monomer of less than 1:4 are Merquat 295 and Merquat 550, which have little or no effect. The amount of nonionic monomer optionally present in the amphoteric polymer of the invention is typically a positive amount up to about 50 mole %, more typically a positive amount up to about 30 mole %, preferably a positive amount up to about 25 mole %, and more preferably a positive amount up to about 15 mole %. As used herein, the term "monomer" is intended to be singular or plural, for example, the term "cationic monomer" could be DMDAAC alone or a mixture of DMDAAC and MAPTAC.

The amphoteric polymers useful herein are available commercially or can be made by polymerizing the desired molar ratios of monomers using standard techniques well known in the polymer art. The weight average molecular weight of the amphoteric polymer is typically between about 250,000 and 7,500,000. For example, Merquat 2001 has a molecular weight of about 1,100,000 and Merquat 3330 has a molecular weight of about 4,000,000.

A preferred amphoteric polymer useful herein is a terpolymer comprising repeating units from acrylic acid, methacrylamidopropyl trimethyl ammonium chloride, and methyl acrylate, and more preferably comprises a reaction product of about 45 mole % acrylic acid, about 45 mole % methacrylamidopropyl trimethyl ammonium chloride, and about 10 mole % methyl acrylate.

Another preferred amphoteric polymer useful herein is a terpolymer comprising Repeating units from acrylic acid, dimethyl diallyl ammonium chloride, and acrylamide, and more preferably comprises a reaction product of about 25 mole % acrylic acid, about 50 mole % dimethyl diallyl ammonium chloride, and about 25 mole % acrylamide.

A difference among polymers that is predictive of the effect on color enhancement, is the relative number of acrylic acid moieties in the molecule. This is summarized in Table I:

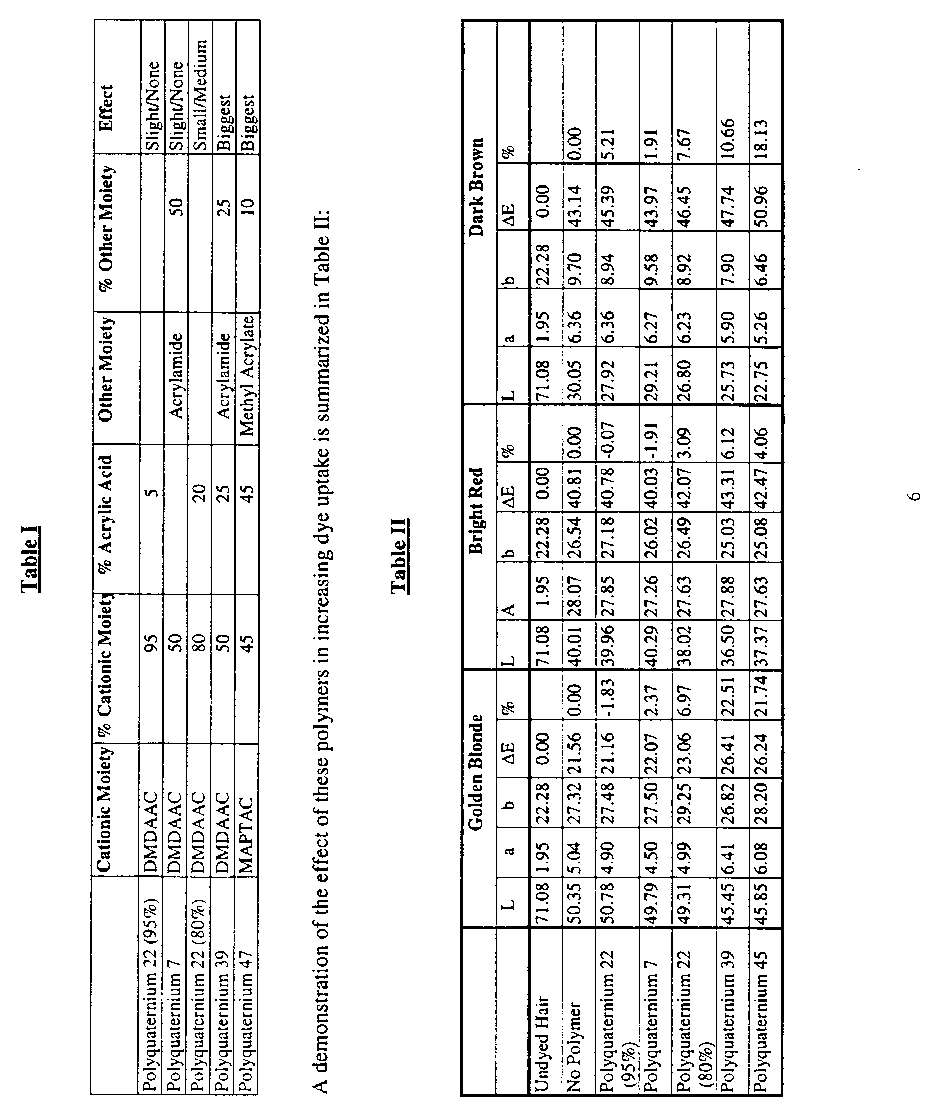

It can be seen from the above tables that quaternized copolymers of acrylic acid and cationic moieties increase the uptake of oxidation dyes significantly when the quantity of acrylic acid is more than about 20 mole % of the polymer. This is a most surprising result considering U.S. Patent RE 33786. Accordingly, the amphoteric polymer useful herein preferably contains acrylic acid in an amount of greater than or equal to about 20 mole %, more preferably greater than 20 mole %, even more preferably greater than or equal to 25 mole %.

For hair coloring compositions of this invention, there may be used one or more suitable primary intermediates, for example:
p-phenylenediamine and its derivatives such as: 2-methyl-p-phenylenediamine, p-phenylenediamine, 2-chloro-p-phenylenediamine, N-phenyl-p-phenylenediamine, N-(2-methoxyethyl)-p-phenylenediamine, N,N-bis(2-hydroxyethyl)-p-phenylenediamine, 2-hydroxymethyl-p-phenylenediamine, 2-(1-hydroxyethyl)-p-phenylenediamine, 2-(2-hydroxyethyl)-p-phenylenediamine, 4,4' diamino-diphenylamine, 2,6-dimethyl-p-phenylenediamine, 2-isopropyl-p-phenylenediamine, N-(2-hydroxypropyl)-p-phenylenediamine, 2-propyl-p-phenylenediamine, 1,3-bis[(N-hydroxyethyl)-N-(4-aminophenyl )amino]-2-propanol, 2-methyl-4-dimethylamino-aniline, 2-methoxy-p-phenylenediamine, 1-2,5-diaminophenyl)-ethane-1,2-diol, 2,3-dimethyl-p-phenylenediamine, 2-(1,2-dihydroxyethyl)-p-phenylenediamine and 2-thiophen-2-yl-benzene-1,4-diamine,
p-aminophenol and its derivatives such as: p-aminophenol, p-methylaminophenol, 3-methyl-4-aminophenol, 2-hydroxymethyl-4-aminophenol, 2-methyl-4-aminophenol, 2-(2' -hydroxyethylaminomethyl)-4-aminophenol, 2-methoxymethyl-4-aminophenol, 5-aminosalicylic acid, 2-(1-hydroxyethyl)-4-aminophenol and 1-(5-amino-2-hydroxyphenyl)-ethane-1,2-diol,
o-aminophenol and its derivatives such as: o-aminophenol, 2,4-diaminophenol, 5-methyl-2-aminophenol, 6-methyl-2-aminophenol, 2-ethylamino-p-cresol, 2-amino-5-acetaminophenol, and 4-methyl-2-aminophenol,
heterocyclic derivatives such as: 2,4,5,6-tetraaminopyrimidine, 4,5-diamino-1-methylpyrazole, 2-dimethylamino-5-aminopyridine, 1-(2-hydroxyethyl)-4,5-diaminopyrazole, 4-hydroxy-2,5,6-triaminopyrimidine, 2-(2-hydroxyethylamino)-6-methoxy-3-aminopyridine and 3-amino-2-methylamino-6-methoxypyridine,

The primary intermediates can be employed in the form of a free base or in the form of an acid additive salt thereof, such as, for example, as a hydrochloride, a hydrobromide, a sulfate or the like.

Suitable couplers include, for example:
phenol derivatives and resorcinol, naphthol and their derivatives such as: 1,7-dihydroxynaphthalene, resorcinol, 4-chlororesorcinol, 1-naphthol, 2-methyl-1-naphthol, 1-acetoxy-2-methylnaphthalene, 1,5-dihydroxynaphthalene, 2,7-dihydroxynaphthalene, hydroquinone, 2-methylresorcinol, 1-hydroxy-6-aminonaphthalene-3-sulfonic acid, 2-isopropyl-5-methylphenol, 1,5-dihydroxy-1,2,3,4-tetrahydronaphthalene, 2-chlororesorcinol, 2,3-dihydroxy-1,4-naphthoquinone and 1-naphthol-4-sulfonic acid, 1,2,3-trihydroxybenzene,
m-phenylenediamines such as: m-phenylenediamine, 2,4-diaminophenoxyethanol, N,N-bis(2-hydroxyethyl)-m-phenylenediamine, 2,6-diaminotoluene, 2-N,N-bis(2-hydroxyethyl)-2,4-diaminophenetole, 1,3-bis(2,4-diaminophenoxy)propane, 1-hydroxyethyl -2,4-diaminobenzene, 2-amino-4-(2-hydroxyethylamino)anisole, 4-(2-aminoethoxy)-1,3-diaminobenzene, 2,4-diaminophenoxyacetic acid, 4,6-bis(2-hydroxyethoxy)-m-phenylenediamine, 2,4-diamino-5-methylphenetole, 2,4-diamino-5-hydroxyethoxy-toluene, 2,4-dimethoxy-1,3-diaminobenzene, 2,6-bis(2-hydroxyethylamino)-toluene, and 3-(2,4-diaminophenoxy)-1-propanol,
m-aminophenols such as: m-aminophenol, 2-hydroxy-4-(carbamoyl-methylamino)toluene, m-carbamoylmethylaminophenol, 6-hydroxybenzomorpholine, 2-hydroxy-4-aminotoluene, 2-hydroxy-4-(2-hydroxyethylamino)toluene, 4,6-dichloro-m-amino-phenol, 2-methyl-m-aminophenol, 2-chloro-6-methyl-m-aminophenol, 2-(2-hydroxyethoxy)-5-aminophenol, 2-chloro-5-trifluoroethylaminophenol, 4-chloro-6-methyl-m-aminophenol, N-cyclopentyl-3-aminophenol, N-hydroxyethyl-4-methoxy-6-methyl-m-aminophenol and 5-amino-4-methoxy-2-methylphenol,
heterocyclic derivatives such as: 1-phenyl-3-methyl-5-pyrazolone, 6-methoxy-8-aminoquinoline, 2,6-dihydroxy-4-methylpyridine, 5-hydroxy-1,4-benzodioxane, 3,4-methylenedioxyphenol, 4-(2-hydroxyethylamino)-1,2-methylenedioxybenzene, 2,6-dihydroxy-3,4-dimethylpyridine, 5-chloro-2,3-dihydroxypyridine, 3,5-diamino-2,6-dimethoxypyridine, 3,4-methylenedioxyaniline, 2,6-bis(2-hydroxyethoxy)-3,5-diaminopyridine, 4-hydroxyindole, 3-amino-5-hydroxy-2,6-dimethoxypyridine, 5,6-dihydroxyindole, 7-hydroxyindole, 5-hydroxyindole, 2-bromo-4,5-methylenedioxyphenol, 6-hydroxyindole, 2-amino-3-hydroxypyridine, 2,6-diaminopyridine, 5-(3,5-diamino-2-pyridyloxy)-1,3-dihydroxypentane, 3-(3,5-diamino-2-pyridyloxy)-2-hydroxypropanol and isatin.

Preferred primary intermediates include:
p-phenylenediamine and its derivatives such as: 2-methyl-p-phenylenediamine, p-phenylenediamine, N-(2-methoxyethyl)-p-phenylenediamine, N,N-bis(2-hydroxyethyl)-p-phenylenediamine, 2-(1-hydroxyethyl)-p-phenylenediamine, 2-(2-hydroxyethyl)-p-phenylenediamine, 1-2,5-diaminophenyl)-ethane-1,2-diol, and 2-(1,2-dihydroxyethyl)-p-phenylenediamine,
p-aminophenol and its derivatives such as: p-aminophenol, p-methylaminophenol, 3-methyl -4-aminophenol, 2-methoxymethyl-4-aminophenol, 2-(1-hydroxyethyl)-4-aminophenol and 1-(5-amino-2-hydroxyphenyl)-ethane-1,2-diol,
o-aminophenol and its derivatives such as: o-aminophenol, 2-ethylamino-p-cresol, 5-methyl-2-aminophenol, 6-methyl-2-aminophenol and 2-amino-5-acetaminophenol, 4-methyl-2-aminophenol,
heterocyclic derivatives such as: 2,4,5,6-tetraaminopyrimidine 4,5-diamino-1-methylpyrazole, 1 -2-hydroxyethyl)-4,5-diaminopyrazole, and 2-dimethylamino-5-aminopyridine.

Preferred couplers include:
phenol derivatives and resorcinol, naphthol and their derivatives such as: 2-methyl-1 -naphthol, 1-acetoxy-2-methylnaphthalene, 1,7-dihydroxynaphthalene, resorcinol, 4-chlororesorcinol, 1-naphthol, 1,5-dihydroxynaphthalene, 2,7-dihydroxynaphthalene, hydroquinone, 2-methylresorcinol and 2-isopropyl-5-methylphenol,
m-phenylenediamines such as: m-phenylenediamine, 2,4-diaminophenoxyethanol, 1 ,3-bis(2,4-diaminophenoxy)propane, 2-amino-4-(2-hydroxyethylamino)anisole and 4,6-bis(2-hydroxyethoxy)-m-phenylenediamine, 3-(2,4-diaminophenoxy)- 1-propanol,
m-aminophenols such as: m-aminophenol, 6-hydroxybenzomorpholine, 2-hydroxy-4-aminotoluene, 2-hydroxy-4-(2-hydroxyethylamino)toluene and 2-methyl-m-aminophenol,
heterocyclic derivatives such as: 1 -phenyl-3-methyl-5-pyrazolone, 3,4-methylenedioxyphenol, 3 ,4-methylenedioxyaniline, 4-hydroxyindole, 5,6-dihydroxyindole, 7-hydroxyindole, 5-hydroxyindole, 6-hydroxyindole, isatin, 2,6-diaminopyridine and 2-amino-3-hydroxypyridine.

Most preferred primary intermediates include:
p-phenylenediamine and its derivatives such as: 2-methyl-p-phenylenediamine, p-phenylenediamine, N,N-bis(2-hydroxyethyl)-p-phenylenediamine and 2-(1-hydroxyethyl)-p-phenylenediamine and 2-(2-hydroxyethyl)-p-phenylenediamine,
p-aminophenol and its derivatives such as: p-aminophenol, p-ethylaminophenol, 3-methyl-4-aminophenol and 1-(5-amino-2-hydroxyphenyl)-ethane-1,2-diol,
o-amino phenol and its derivatives such as: o-aminophenol, 2-ethylamino-p-cresol, 5-methyl-2-aminophenol, 6-methyl-2-aminophenol and 2-amino-5-acetaminophenol,
heterocyclic derivatives such as: 2,4,5,6-tetraaminopyrimidine, 1-(2-hydroxyethyl)-4,5-diaminopyrazole.

Most preferred couplers include:
Phenol derivatives and resorcinol, naphthol and their derivatives such as: 2-methyl- 1-naphthol, 1 -acetoxy-2-methylnaphthalene, resorcinol, 4-chlororesorcinol, 1-naphthol and 2-methylresorcinol,
m-phenylenediamines such as: 2,4-diaminophenoxyethanol, 2-amino-4-(2-hydroxyethylamino)anisole and 4,6-bis(2-hydroxyethoxy)-m-phenylenediamine, and 3-(2,4-diaminophenoxy)- 1-propanol,
m-aminophenols such as: m-aminophenol, 6-hydroxybenzomorpholine, 2-hydroxy-4-aminotoluene, 2-hydroxy-4-(2-hydroxyethylamino)toluene and 2-methyl-m-aminophenol,
heterocyclic derivatives such as: 1-phenyl-3-methyl-5-pyrazolone, 2-amino-3-hydroxypyridine and 6-hydroxyindole.

The hair coloring compositions of this invention will generally contain one or more primary intermediates in an effective coloring amount, generally in an amount of from about 0.01 to about 5.0 weight percent, preferebly 0.05 to 3.5 weight percent. The coupler(s) will generally be present in an amount of from about 0.01 to about 5.0 weight percent, preferably 0.05 to 3.5 weight percent. The molar ratio of primary intermediate to coupler will generally range from about 5:1 to about 1:5 and be employed in any suitable carrier or vehicle, generally an aqueous or hydroalcoholic solution, preferably an aqueous solution. The carrier or vehicle will generally comprise up to about 40 weight percent.

Other conventional agents often employed in hair coloring compositions may be employed in the dye component or in the developer component of the invention. These include, for example, fragrances, coloring agents and chelating agents.

Antioxidants, such as sodium sulfite, erythorbic acid and ascorbic acid, may also be included to inhibit premature oxidation.

A significant feature of the present invention is the discovery of a limited class of conditioning, amphoteric, quaternized polymers can be added to an oxidative hair dye to increase the amount of color that is deposited when the oxidative hair dye is used to color hair.

The total amount of amphoteric polymer in the formulation is typically between 0.05 and 10% and preferably between about 0.25 and 2.5%, based on the total weight of the composition.

Unless otherwise indicated all weight percentages recited herein are based on the total weight of the composition, i.e., the concentration in the total formulation as it is applied to the hair.

The oxidative dye compositions of the invention preferably meet at least the following conditions:
1. They must provide a range of color shades that are acceptable to practitioners of hair coloring.
2. The couplers and primary intermediates must be properly dispersed to ensure that they rapidly penetrate into the hair.
3. The dye component should have the necessary properties so that when it is mixed with the developer, the resulting mixture has the desired rheological properties. The mixture preferably is thin enough to be able to spread onto the hair, but thick enough to stay in place during the color development period. If thickened, the mixture should also be readily rinsable from the hair with water.
4. The formulations should not be irritating to the scalp.

In order to meet conditions 2 and 3 above, mixtures of surfactants, organic solvents and thickeners, are included in all commercial hair dye preparations, and are preferably present in the compositions of the invention.

A wide variety of surfactants and surfactant mixtures may be employed in the practice of this invention. Several typically useful surfactants are set forth below. As with all categories of components recited herein, individual members of a given category may be used singly or in blends containing at least two of them. The amount of surfactant in the dye component of the invention is typically about 1 to about 30 weight %, preferably about 2.5 to about 10 weight %. Useful surfactants include amphoteric surfactants such as betaines, sultaines, glycinates, proprionates; alkylpolyglycosides; fatty acid soaps such as alkanolamine, alkali metal or alkaline earth metal salts of a carboxylic acid containing from about 11 to 19 carbon atoms; higher alkylbenzene sulfonates; alkylnaphthalenesulfonates; sulfonated esters of alcohols and polybasic acids; taurates; fatty alcohol sulfates; sulfates of branched chain or secondary alcohols; alkyldimethylbenzylammonium chlorides, salts of fatty acids or fatty acid mixtures. Illustrative of specific surfactants that can be used are sodium lauryl sulfate; polyoxyethylene lauryl ether: myristyl sulfate; glyceryl monostearate; triethanolamine oleate, sodium salt of palmitic methyl taurine; cetyl pyridinium chloride; lauryl sulfonate; myristyl sulfonate; lauric diethanolamide; polyoxyethylene stearate; ethoxylated oleoyl diethanolamide; stearyldimethyl benzyl ammonium chloride; dodecylbenzene sodium sulfonate; triethanolamine salt of p-dodecylbenzene sulfonate; nonylnaphthalene sodium sulfonate; dioctyl sodium sulfosuccinate; sodium N-methyl-N-oleoyl taurate; oleic acid ester of sodium isothionate; sodium dodecyl sulfate; the sodium salt of 3-diethyl tridecanol-6-sulfate and the like. Preferred surfactants useful herein are nonoxynol-2, nonoxynol-4, nonoxynol-9, octoxynol-1, laureth-4, laureth-23, oleth-4, oleth-21, sodium lauryl sulfate, sodium lauryl ether sulfate, sodium dodecyl benzene sulfonate, and sodium cocamido propylbetaine, as well as certain cationic surfactants that will be described under conditioners.

Typical organic solvents used in hair color formulations include ethyl alcohol, isopropyl alcohol, carbitol, propylene glycol and hexylene glycol, and such solvents are optionally present in the compositions of the invention. The amount of organic solvent is typically varied from about 2 weight % to about 15 weight %.

Typical thickeners useful herein include the surfactants described above, lauric acid diethanolamide, cocamide DEA and polyacrylic acid derivatives. The amount of thickener is typically about 0.25 to about 20 weight %, preferably about 2 to about 10 weight %.

Because treatment with alkaline hydrogen peroxide can damage the surface of the hair, making it difficult to comb and feel rough, other conditioners are frequently added to a hair dye formulations, and are optionally present in the compositions of the invention. Conditioners are usually cationic surfactants, cationic polymers or aminofunctional silicones. Typical other conditioners that are useful herein (i.e., other than the amphoteric polymer) include soyatrimmonium chloride, dicetyldimonnium chloride, behenyltrimmonium Polyquaternium 22 and amodimethicone. The amount of other conditioner can be a positive amount and is typically about 0 to about 5 weight %, preferably about 0 to about 2 weight %.

Generally, the addition of thickeners and surfactants, including those surfactants used as conditioners, to haircolor formulations makes the dyeing less efficient as was explained in U.S. Patent RE33786. Addition of such materials to a formulation requires increases in all the dyes in the formula to obtain the same shade. The conditioning, quaternized, amphoteric polymers required in the dye component of the present invention results in enhanced dye color which is surprising in view of the general teachings of the prior art.

Any of a wide variety of alkalizing agents may be employed in the practice of this invention. Because of its freedom from toxicity over a wide concentration range and its economy, ammonium hydroxide is an acceptable alkalizing agent. However, any other compatible ammonia derivative can be used in place of, or together with, ammonium hydroxide to effect the desired alkalinity. For example, an alkylamine such as ethylamine, or triethylamine; or alkanolamines, such as ethanolamine, diethanolamine, aminomethyl propanol, aminomethyl propanediol and trishydroxymethyl aminomethane may be employed. Likewise, any other of the organic or inorganic alkalizing agents may be used, such as sodium or potassium hydroxide, sodium or potassium carbonate, sodium phosphate, sodium hydrogen phosphate, sodium silicate, guanidine hydroxide and the like. The preferred alkaline reagents are ammonium hydroxide, sodium carbonate and ethanolamine.

When the alkaline reagents listed above, or their equivalents, are employed at a concentration of from about 0.5% to 10%, the pH of the dye component will be from about 8 to 10.5.

Of course, water is usually present in the final formulation as it is applied to the hair in significant amounts, e.g., about 50 to about 90 weight %, preferably about 65 to about 80 weight %.

Although they are not essential, buffering agents may be employed to stabilize the pH of the dye component during storage. Typically useful buffers include ammonium and alkali metal phosphates, bicarbonates, carbonates and, to a lesser extent, borates. Also suitable are amino buffers such as, N-[2-hydroxyethyl]-piperazine-N¹-[2-ethanesulfonic acid] (HEPES), N-[2-acetamido]-2-aminoethane sulfonic acid (ACES), tris[hydroxymethyl] aminomethane (TRIS) and N-tris[hydroxymethyl]-methyl-3-aminopropane sulfonic acid (TAPS). The ammonium and alkali metal carbonates are also suitable. The preferred buffers are TRIS, sodium and potassium carbonate, bicarbonate and phosphate.

The dye component and the developer component can be combined by mixing them directly on the hair of the user. If they are mixed on the hair, adding the dye component first is preferable. It is preferred to mix them in a mixing vessel for subsequent application to the hair.

The present invention also encompasses a kit comprising at least two containers, one containing the dye component, the other the developer component, the two when mixed forming a composition of the present invention.

The method of the invention comprises applying the oxidative dye composition of the present invention to the hair to be colored and allowing it to remain in contact with the hair until the desired hair color has been attained after which the composition is removed from the hair by rinsing.

The following examples are to illustrate the invention, but should not be interpreted as a limitation thereon.

### EXAMPLES

Some examples of compositions that are encompassed by this patent are given in Table III. In Table III, the weight percentages for Polyquaternium -47 and Polyquaternium -39 are based on the form of the material as provided by the supplier; Polquaternium -47 is 21% active and Polyquaternium -39 is 9% active.

**Table III**

| **WEIGHT PERCENT IN FORMULA** | | | | | | | | | |
|---|---|---|---|---|---|---|---|---|---|
| **NAME** | 1 | 2 | 3 | 4 | 5 | 6 | 7 | 8 | 9 |
| M-Aminophenol | 0 | 0.1 | 0.4 | 0.8 | 0.01 | 0.005 | 0 | 0 | 0 |
| 1-Naphthol | 0.1 | 0 | 0.2 | 0 | 0.15 | 0.2 | 0.13 | 0.6 | 0 |
| Resorcinol | 1 | 0.078 | 0.2 | 1.1 | 0.05 | 0 | 0.2 | 0.12 | 0.13 |
| P-Phenylenediamine | 1.4 | 0.1 | 0.3 | 1.1 | 0.03 | 0 | 0 | 0 | 0.25 |
| P-Aminophenol | 0.03 | 0.24 | 0.1 | 0.4 | 0.01 | 0.5 | 0.1 | 0 1 | 0.32 |
| 28% Ammonium | 8 | | 4 | 7 | | 2 | 9 | 10 | 8 |
| Hydroxide | | | | | | | | | |
| Monoethanolamine | | 6 | | | 9 | 4 | | | |
| 2-Methylresorcinol | 0 | 0.429 | 0.429 | 0.429 | 0.343 | 0.34 | 068 | 0 | 0 |
| 4 Amino-2-Hydroxytoluene | 0 | 0.05 | 0.05 | 0.05 | 0.04 | 0 | 0 | 0 | 1.9 |
| Behentrimonium Chloride | | | | 2 | | | | | |
| C12-15 Pareth-3 | | 2 | 4.5 | | 4.5 | | 6 | 4.5 | 4 |
| Polyquaternium - 47 | 6 | 3 | | | | 4 | 4 | 5 | |
| Propylene Glycol | | 4 | | 1 | 5 | 2 | 6 | | |
| Polyquaternium-39 | | | 4 | 4 | 6 | | | | 2 |
| Cetyl Alcohol | 0.2 | 1 | 1 | | 1 | 1 | 1 | 1 | 1 |
| Stearyl Alcohol | | 0.2 | | 0.3 | | | | | |
| Oleic Acid | 7 | | 2 | 3 | | 0.5 | 4 | 13 | 6 |
| Citric Acid | | 3 | | | 4 | | | | |
| Nonoxynol-4 | 5 | | | | | | | | |
| Octoxynol-1 | 10 | | | 6 | | | | | |
| Ethoxydiglycol | 4 | | | 6 | | | | | |
| Isopropyl Alcohol | 12 | | | 4 | | 5 | | | |
| Fragrance | 0.1 | 0.1 | | 0.4 | | 0.2 | | | |
| Steareth-21 | 0.4 | 1 | 2 | | 1 | 0.8 | 1 | 1 | 0.9 |
| C11-15 Pareth-9 | | 1.1 | 2 | | 0.5 | 1.3 | 1 | 1.5 | 2 |
| Water | 44.77 | 77.603 | 78.821 | 62.421 | 68.367 | 78.155 | 66.89 | 63.18 | 73.5 |

Table IV sets forth the suitable combinations of primary intermediates and couplers that may be used in the hair dye compositions of the present invention containing the amphoteric polymers disclosed herein. The primary intermediates and couplers are present in amounts effective to dye hair as disclosed herein and the compositions are used in accordance with the procedures for doing so set forth herein.

With the foregoing description of the invention, those skilled in the art will appreciate that modifications may be made to the invention without departing from the spirit thereof. Therefore, it is not intended that the scope of the invention be limited to the specific embodiments illustrated and described.

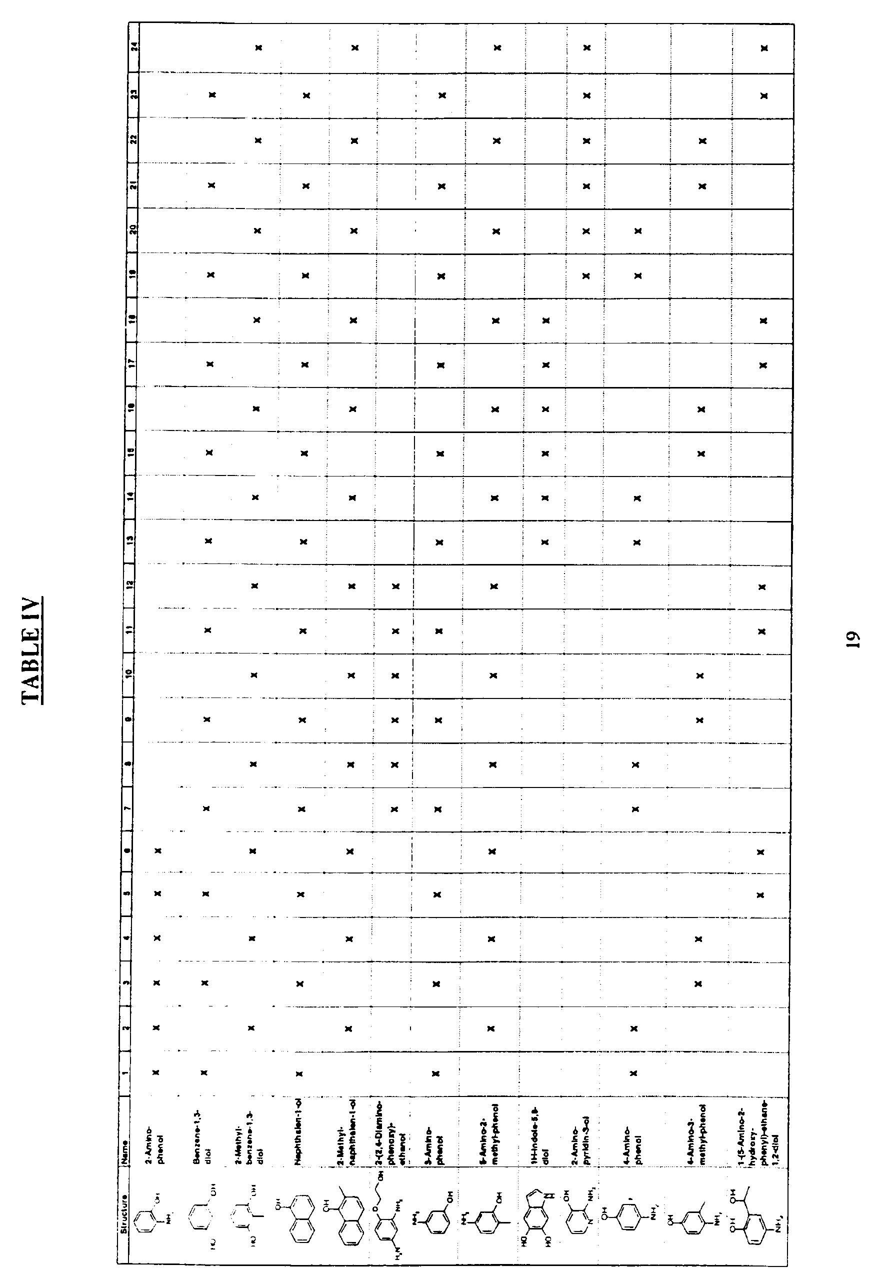

## Claims

1. A dye component of a hair coloring composition comprising a mixture of:
(A) an amphoteric polymer comprising repeating units from:
(a) acrylic acid, and
(b) a cationic monomer selected from the group consisting of: methacrylamidopropyl trimethyl ammonium chloride, dimethyl diallyl ammonium chloride; and a mixture thereof, wherein the molar ratio of (a):(b) is 1:3 or greater;
(B) an oxidative dye primary intermediate; and
(C) an oxidative dye coupler.

2. The composition of Claim 1 wherein said amphoteric polymer further comprises repeating units from
(c) a nonionic monomer.

3. The composition of Claim 2 wherein said nonionic monomer is selected from the group consisting of acrylamide, one or more acrylic acid esters, and a mixture thereof.

4. The composition of Claim 3 wherein said nonionic monomer is selected from the group consisting of acrylamide, methyl methacrylate, and a mixture thereof.

5. The composition of Claim 1 wherein said amphoteric polymer is a terpolymer.

6. The composition of Claim 5 wherein said terpolymer has a molecular weight between about 250,000 to about 7,500,000.

7. The composition of Claim 1 wherein the molar ratio of (a): (b) is 1:2 or greater.

8. The composition of Claim 1 wherein said amphoteric polymer is a terpolymer comprising repeating units from acrylic acid, methacrylamidopropyl trimethyl ammonium chloride, and methyl acrylate.

9. The composition of Claim 8 wherein said terpolymer comprises a reaction product of about 45 mole % acrylic acid, about 45 mole % methacrylamidopropyl trimethyl ammonium chloride, and about 10 mole % methyl acrylate.

10. The composition of Claim 1 wherein said amphoteric polymer is terpolymer comprising repeating units from acrylic acid, dimethyl diallyl ammonium chloride, and acrylamide.

11. The composition of Claim 10 wherein said terpolymer is a reaction product of about 25 mole % acrylic acid, about 50 mole % dimethyl diallyl ammonium chloride, and about 25 mole % acrylamide.

12. The composition of Claim 1 wherein said oxidative dye primary intermediate is selected from the group consisting of p-phenylenediamine, p-toluenediamine, N,N-Bis(2-hydroxyethyl)-p-phenylenediamine, p-amino-phenol, 2-chloro-p-phenylenediamine, 2-methoxy-p-phenylenediamine, 4-amino-m-cresol, 2-hydroxyethyl-p-phenylenediamine, 1 -(2,5-diaminophenyl)- 1-2 ethane- 1 ,2-diol, 1 -(5-amino-2-hydroxyphenyl)ethane-1,2-diol and mixtures thereof, and said oxidative dye coupler is selected from the group consisting of resorcinol, 1-naphthol, 2-methylnaphthol, 2-methyl-resorcinol, m-aminophenol, 2,4-diaminophenolethanol, 5-methyl-3-aminophenol, 4-methyl-3-aminophenol, m-phenylenediamine, and mixtures thereof.

13. The composition of Claim 1 comprising about 0.05 to about 10 weight % of component (A) about to about weight % of compound (B), and about to about weight % of compound (C).

14. The composition of Claim 1 comprising about 0.25 to about 2.5 active weight % of component (A) about .01 to about 5 weight % of compound (B), and about .01 to about 5.0 weight % of compound (C), based on total composition weight.

15. The composition of Claim 1 further comprising about 1 to about 30 weight % of a surfactant.

16. The composition of Claim 15 wherein said surfactant is selected from the group consisting of nonoxynol-2, nonoxynol-4, nonoxynol-9, octoxynol-1, laureth-4, laureth-23, oleth-4, oleth-21, sodium lauryl sulfate, sodium lauryl ether sulfate, sodium dodecyl benzene sulfonate, and sodium cocamido propylbetaine.

17. The composition of Claim 1 further comprising about 0.5 to about 10 weight % of an alkalizing agent, based on total composition weight.

18. The composition of Claim 1 further comprising about 0.2 to about 15 weight % of a buffering agent.

19. The composition of Claim 1 further comprising a developer component.

20. A kit comprising at least two containers, one container containing the dye component of Claim 1 and another container containing a developer component.

21. A method comprising applying the oxidative dye composition of Claim 19 to hair, allowing the composition to remain in contact with the hair until a desired hair color has been attained, and rinsing excess dye composition from the hair.
